# EUROPEAN PATENT SPECIFICATION

(11) **EP 2 637 643 B1**
(45) Date of publication and mention of the grant of the patent: **17.12.2014**
(21) Application number: 11779654.0
(22) Date of filing: 07.11.2011
(51) Int. Cl.: A61K 9/16, A61K 31/4035, A61P 31/14

(54) **PHARMACEUTICAL COMPOSITION FOR TREATING HCV INFECTIONS**
PHARMAZEUTISCHE ZUSAMMENSETZUNG ZUR BEHANDLUNG VON HCV-INFEKTIONEN
COMPOSITION PHARMACEUTIQUE POUR LE TRAITEMENT DES INFECTIONS PAR LE VHC

(30) Priority: 09.11.2010 EP 10190461
(43) Date of publication of application: 18.09.2013
(73) Proprietor: F.Hoffmann-La Roche AG, 4070 Basel (CH)
(72) Inventor: LEIMINER, Andreas, 79639 Grenzach-Wyhlen (DE); LINDENSTRUTH, Kai, 10557 Berlin (DE); MILLER, Dave Alan, Livingston, NJ 07039 (US); SCHEUBEL, Emmanuel, F-68510 Sierentz (FR); SHAH, Navnit Hargovindas, Clifton, NJ 07012 (US)
(74) Representative: Halbig, Dirk
(86) International application number: PCT/EP2011/069492
(87) International publication number: WO 2012/062685

(56) References cited:
- WO-A1-2007/082809
- WO-A1-2010/122087
- PASSERINI N ET AL: "Evaluation of melt granulation and ultrasonic spray congealing as techniques to enhance the dissolution of praziquantel", INTERNATIONAL JOURNAL OF PHARMACEUTICS, ELSEVIER BV, NL, vol. 318, no. 1-2, 2 August 2006 (2006-08-02), pages 92-102, XP025112992, ISSN: 0378-5173, DOI: DOI:10.1016/J.IJPHARM.2006.03.028 [retrieved on 2006-08-02]

## Description

The present invention provides novel formulations for the treatment of HCV infections containing 4-fluoro-1,3-dihydro-isoindole-2-carboxylic acid (Z)-(1S,4R,6S,14S,18R)-14-tert-butoxycarbonylamino-4-cyclopropanesulfonylaminocarbonyl-2,15-dioxo-3,16-diaza-tricyclo[14.3.0.0^{4,6}]nonadec-7-en-18-yl ester hereinafter referred to as compound I and pharmaceutically acceptable salts thereof. Compound I has activity as an antiviral agent.

Compound I is a peptide analog known for the treatment of HCV infection. The compound can be used alone or in combination with an amount of one or more additional antiviral agent(s), effective to achieve a sustained viral response in the patient. The compound I inhibits the enzymatic activity of a hepatitis virus C (HCV) protease NS3. Such compounds are described in WO 2005/037214.

Compound I is available in both crystalline and amorphous forms and has pH-dependent physicochemical properties, in particular solubility and permeability, in the physiological range, Owing to solubility and permeability limitations, compound I is considered a Biopharmaceutical Classification System Class 4 compound (solubility and permeability limited oral absorption).

Weak acids with pH-dependent physicochemical properties present unique challenges to the formulation scientist. For drugs with dissolution rate limited solubility and bioavailability it becomes a significant challenge. The general approaches used to improve the bioavailability includes reducing the particle size of the drug, use of co-solvents or complexing agents, dispersing the drug in hydrophilic matrices, using lipid based drug delivery systems such as self-emulsifying drug delivery systems, microemulsions, micellar systems, solid and molecular dispersion and has been widely discussed, e.g., Choi et al., Drug Dev. Ind. Pharm., vol 29(10), 1085-1094, 2003; Yueksel et al., Eur. J. Pharm. and Biopharm., vol 56(3), 453-459, 2003 and U.S. Pat. No. 6,632,455.

The bioavailability challenge presented by compound I is not simply the result of low solubility but specifically due to its unique tendency toward cohesive particle interactions in aqueous media. When the crystalline salt form of compound I is placed in acidic media it rapidly dissociates forming the amorphous free acid. Owing to hydrophobicity, these amorphous particles aggregate to minimize surface contact with the aqueous media. This loose association rapidly leads to particle agglomeration and the formation of larger particulate structures. This phenomena results in a marked reduction in the surface area of compound I in the aqueous environment and consequently a decrease in dissolution rate.

It is this particle interaction in aqueous media that primarily limits the bioavailability of compound I. There is therefore a need for a formulation approach which can overcome this problem in order to improve oral absorption and therapeutic efficacy of compound I.

Surprisingly, it was found by the present inventors that a granular pharmaceutical composition comprising compound I drug particles and at least one poloxamer overcomes the afore-described disadvantages in the art and provides for an improved dispersability of compound I, which ultimately results in an enhanced pharmacokinetic performance, i.e. greater and less variable oral absorption of compound I. The present invention provides thus a solid pharmaceutical composition of compound I with improved pharmacokinetic performance, i.e., enhanced bioavailability, reduced variability, and reduced food effect. The dissolution rate of compound I in aqueous media from poloxamer containing formulations is surprisingly independent of the drug particle size. This is contrary to the previous understanding in the art that dissolution rate and bioavailability of poorly water soluble drugs from crystalline particulate dispersions in a poloxamer or similar hydrophilic matrices are strongly dependent on API particle size.

Manufacturing technologies such as wet or dry granulation, fluid bed granulation, hot melt extrusion, spray drying, spray congealing, solvent evaporation and high shear granulation are useful approaches to obtain the granular pharmaceutical composition according to the present invention by intimate mixing. In one embodiment of the present invention, the granular pharmaceutical composition is manufactured by means of hot melt extrusion. Surprisingly, it has been found that hot melt extrusion resolves a number of manufacturing and powder flow difficulties which are typical for a granulation process of aggregating flocculent and poorly compressible powders like compound I drug substance. Hot melt extrusion achieves optimal results with respect to manufacturability, stability, bioavailability, and patient convenience of the granular pharmaceutical composition according to the present invention.

As used herein, the following terms have the meanings set out below.

The term "API" refers to the active pharmaceutically active ingredient.

The term "excipients" refers to an inactive substance used as a carrier for an active pharmaceutical ingredient. Excipients may be used to aid in the absorption of the active pharmaceutical ingredient, to bulk up formulations to aid in the manufacturing process, or to help stabilize the active pharmaceutical ingredient. In order to maximize the physical characteristics of the tablets the formulation may further contain other pharmaceutically acceptable excipients such as antiadherents, binders, filler/diluents, disintegrants, stabilizers, compression aids, lubricants, granulation aids, flow aids, and the like. The membrane coating may further contain other coating excipients such as opacifiers, pigments, colorants and the like. The choice of such materials and the amounts to be utilized are considered to be within the art.

The term "diluent" or "filler" as used herein refers to an inert excipient added to adjust the bulk in order to produce a size practical for compression. Common diluents include dicalcium phosphate, calcium sulfate, lactose, cellulose, kaolin, mannitol, sodium chloride starch and powdered sugar. Diluents such as mannitol, lactose, sorbitol, sucrose and inositol in sufficient quantities aid disintegration of the tablet and are frequently used in chewable tablets. Microcrystalline cellulose (AVICEL^{®}) has been used as an excipient in wet granulation and direct compression formulations.

The term "poloxamer" denotes non-ionic triblock copolymers composed of a central hydrophobic chain of poly(propylene oxide) (PPO) flanked by two hydrophilic chains of poly(ethylene oxide) (PEO), each PPO or PEO chain can be of different molecular weights. Poloxamers are also known by the trade name Pluronics. Particular Poloxamer is Poloxamer 188, a poloxamer wherein the PPO chain has a molecular mass of 1800 g/mol and a PEO content of 80% (w/w). Poloxamers are available in wide range of molecular weights, melting points and hydrophilicity and are commonly used in the pharmaceutical formulations as wetting agents to improve the bioavailability.

Poloxamer 188 (Lutrol F68.RTM.) is a block copolymer of ethylene oxide and propylene oxide and is listed in the NF monograph as poloxamer 188. Poloxamers are available in wide range of molecular weights, melting points and hydrophilicity and are commonly used in the pharmaceutical formulations as wetting agents to improve the bioavailability. They are supplied by BASF (NJ, USA). The Lutrol F68.RTM. used in this invention has molecular weight in the range of 8400 daltons, melting point of 52.degree.-54.degree. C. and HLB (hydrophilic-lipophilic balance) of 18-29 and the average particle size ranging from 1 micron to 500 microns.

The term "binder" as used herein refers to an excipient added to impart cohesive qualities to the powder which allows the compressed tablet to retain its integrity. Materials commonly used as binders include starch, gelatin and sugars such as sucrose, glucose, dextrose, molasses and lactose. Natural and synthetic gums including acacia, sodium alginate, panwar gum, ghatti gum, carboxymethyl cellulose, methyl cellulose, polyvinylpyrrolidone, ethyl cellulose and hypromellose have also be used binders in some formulations.

The term "lubricants" as used herein refers to an excipient added to prevent adhesion of the tablet material to the surface of dyes and punches. Commonly used lubricants include talc, magnesium stearate, calcium stearate, stearic acid, hydrogenated vegetable oils and PEG. Water soluble lubricants include sodium benzoate, mixtures of sodium benzoate and sodium acetate, sodium chloride, leucine and Carbowax 4000.

The term "glidant" as used herein refers to an excipient added to improve the flow characteristics of the tablet powder. Colloidal silicon dioxide (AEROSIL^{®}) is a common glidant. Talc may serve as a combined lubricant/glidant.

The term "disintegrant" as used herein refers to a excipient added to facilitate breakup or disintegrate after administration. Dried and powdered corn starch or potato starch are popular disintegrants. They have a high affinity for water and swell when moistened leading to rupture of the tablet. A group of materials known as super-disintegrants include croscarmellose sodium, a cross-linked cellulose, crosprovidone, a cross-linked polymer and sodium starch glycolate, a cross-linked starch. Crosprovidone (POLYPLASDONE^{®}) is a synthetic, insoluble, but rapidly swellable cross-linked N-vinyl-pyrrolidone homopolymer.

The term "pharmaceutically acceptable," such as pharmaceutically acceptable carrier, excipient, etc., means pharmacologically acceptable and substantially non-toxic to the subject to which the particular compound is administered.

The term "pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Sample base-addition salts include those derived from ammonium, potassium, sodium, and quaternary ammonium hydroxides, such as for example, tetramethylammonium hydroxide. Chemical modification of a pharmaceutical compound (i.e., drug) into a salt is a technique well known to pharmaceutical chemists to obtain improved physical and chemical stability, hygroscopicity, and solubility of compounds. *See, e.g.,* H. Ansel et. al., Pharmaceutical Dosage Forms and Drug Delivery Systems (6th Ed. 1995) at pp. 196 and 1456-1457.

The term "extragranular" refers to the tablet ingredients added to a hot melt or wet granular mixture *(i.e.,* the first granular component) of compound I and a binder. For the sake of clarity a tablet or capsule, however, can contain more than one granular component.

The term "sustained viral response" (SVR; also referred to as a"sustained response"or a"durable response"), as used herein, refers to the response of an individual to a treatment regimen for HCV infection, in terms of serum HCV titer. Generally, a"sustained viral response"refers to no detectable HCV RNA (e. g. , less than about 500, less than about 200, or less than about 100 genome copies per milliliter serum) found in the patient's serum for a period of at least about one month, at least about two months, at least about three months, at least about four months, at least about five months, or at least about six months following cessation of treatment.

The term "hot melt extrusion" or "HME" refers to a thermal processing that has been adopted from the plastics industry to manufacture matrix systems for pharmaceutical purposes. The therapeutic compound is usually included as a powder or granules into the formulation and dispersed in a molten thermoplastic carrier such as waxes or polymers during processing. The thermal processes involve elevated temperatures and the application of shear forces. Upon solidification, the material may be ground into powders for post-processing or cut into tablets, mini-rods or cylinders for post spheronization.

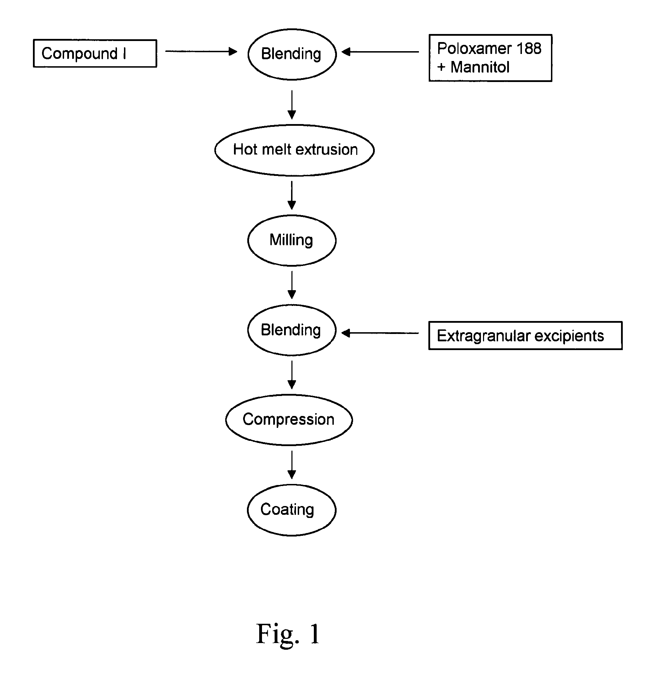

Fig. 1 schematically describes the manufacturing process of the granular pharmaceutical composition according to the present invention comprising the compound I and at least one poloxamer, wherein compound I, the at least one poloxamer and optionally a water soluble filler are combined by hot melt extrusion processing, milled and sieved prior to inclusion with other ingredients (excipients) required for the final oral dosage form.

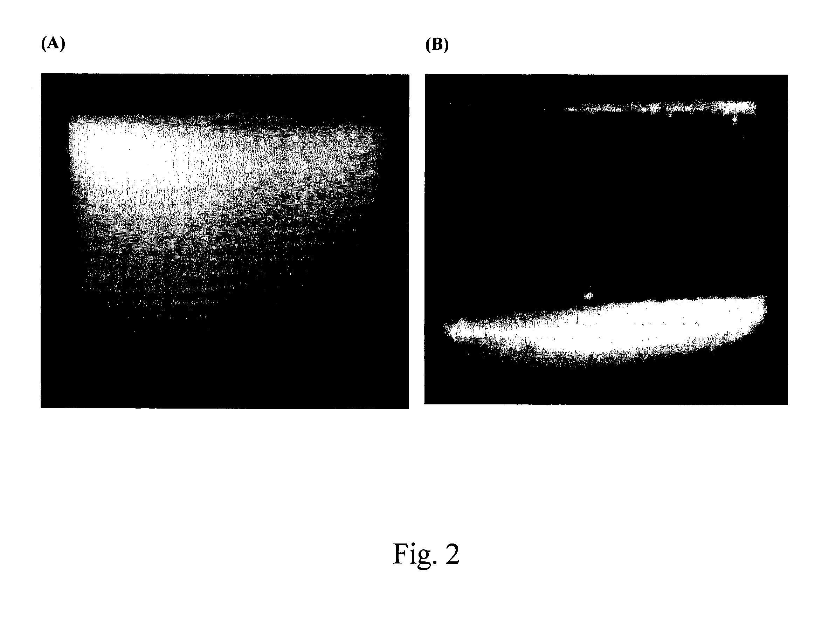

Fig. 2(A) relates to Compound I-poloxamer 188 HME granules and Fig. 2(B) to Compound I-PEG 8000 HME granules both suspended in 0.1 N HCl for two hours. Fig. 2(A) and Fig. 2(B) demonstrate the efficacy of poloxamer 188 with regard to maintaining a fine suspension of compound I particles in acidic media. Maintaining a finely dispersed suspension of compound I in acid is critical for improving oral absorption of compound I as it ensures that the drug is in a rapidly dissolving form when it reaches the intestinal tract.

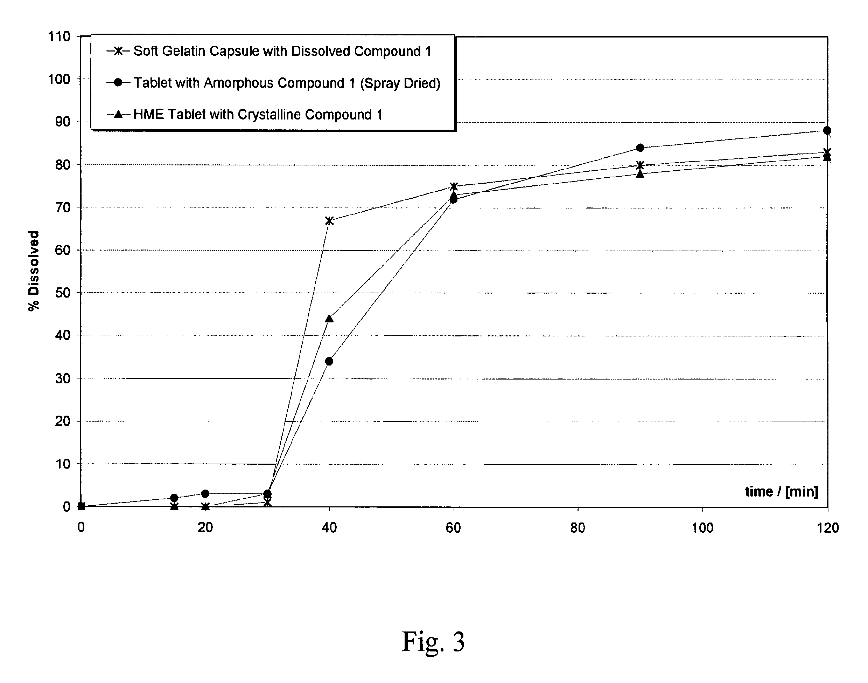

Fig. 3 reflects the comparative dissolution performance of: (1) the hot-melt extruded tablets produced according to Example 1 (shown as triangles), (2) soft gelatin capsule containing a solution of compound I (shown as stars), and (3) a tablet containing an amorphous dispersion of compound I produced by spray drying (shown as circles).

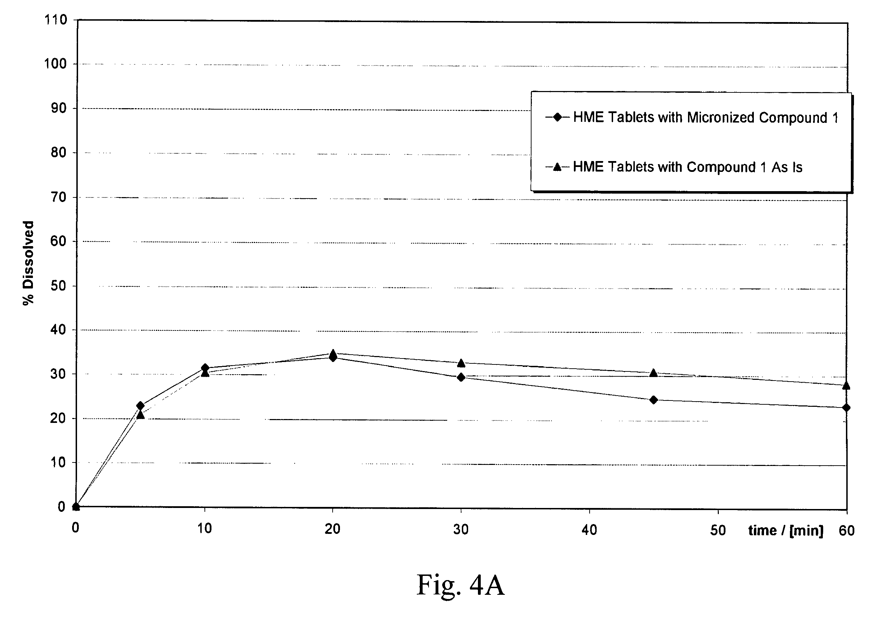

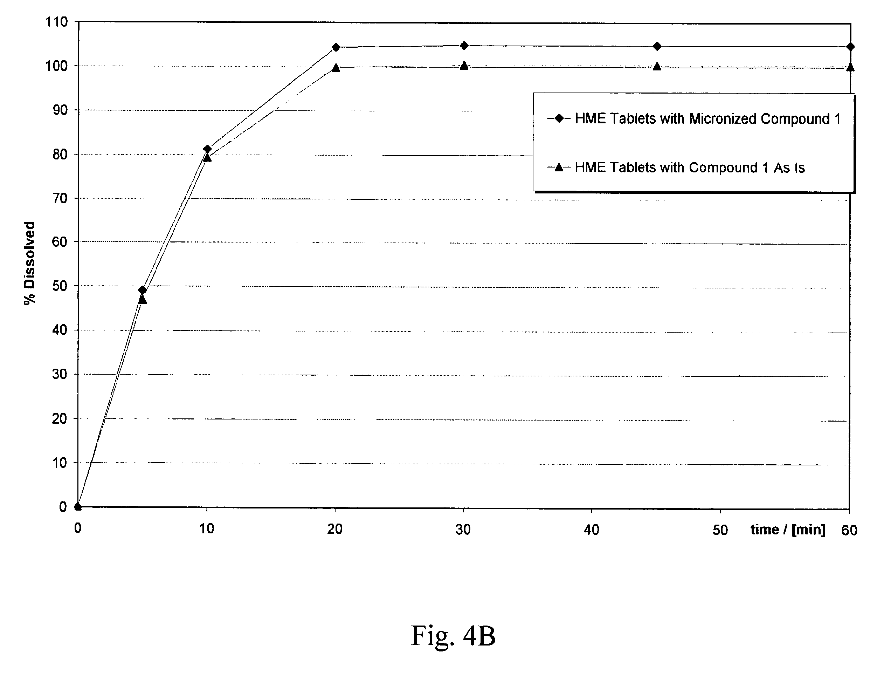

Fig. 4 shows the results of dissolution tests comparing the release of compound I in Fig. 4(A) at pH 5.0 and Fig. 4(B) at pH 7.5 acetate buffer from tablets produced according to Example 2 containing micronized and as-is forms of compound I.

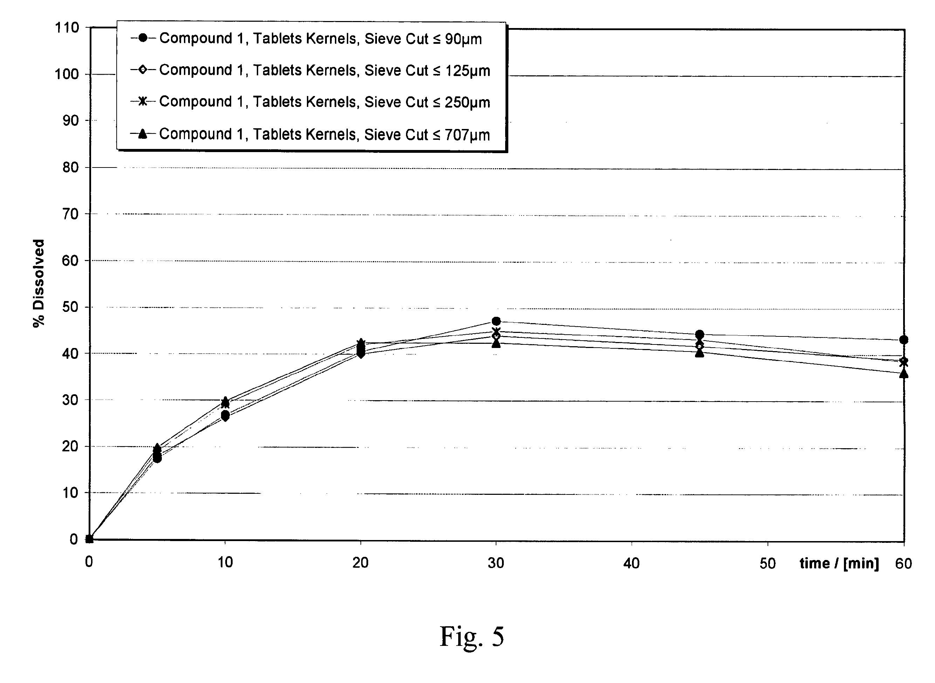

Fig. 5 shows the results of dissolution tests for compound I tablets containing hot melt extruded granules of varying size.

In a first embodiment, the present invention relates to a granular pharmaceutical composition comprising a compound of formula (I) 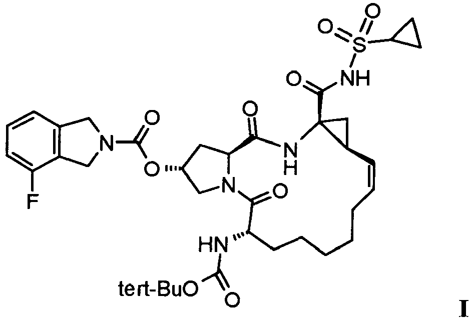 (also referred to as compound I) or a pharmaceutically acceptable salt thereof and at least one poloxamer, wherein the at least one poloxamer is poloxamer 188. Compound I can either be present in a crystalline or amorphous state.

The granular pharmaceutical composition according to the present invention preferably comprises from 20 to 50% wt/wt of the compound I and from 20 to 40 % wt/wt of poloxamer 188.

In a further alternative embodiment, the granular pharmaceutical composition according to the present invention further comprises an intragranular filler like dicalcium phosphate, calcium sulfate, lactose, cellulose, kaolin, mannitol, sodium chloride starch and powdered sugar. Preferably, mannitol is added as an intragranular filler in an amount of up to 80 % wt/wt, and even more preferably in an amount of up to 40 % wt/wt. A preferred embodiment of the present invention concerns a granular pharmaceutical composition comprising 40% wt/wt of compound I, 23% wt/wt of poloxamer 188 and 37% wt/wt of mannitol.

In yet another alternative embodiment, the granular pharmaceutical composition according to the present invention is a binary composition consisting of a compound of formula (I) from 20 to 80% wt/wt and a poloxamer from 20 to 80% wt/wt. Preferably, said binary composition consists of a compound of formula (I) from 40 to 60% wt/wt and a poloxamer from 40 to 60% wt/wt.

The granular pharmaceutical composition according to the present invention can be obtained by a hot melt extrusion process. The present invention therefore also provides for a method for the preparation of granular pharmaceutical compositions comprising compound I and at least one poloxamer by HME. Hot-melt extrusion commonly uses single or twin screw extruders of varying sizes and with one or several temperature zones. The energy input by the extrusion system, either from external heat supplied to the different temperature zones or from the mechanical energy of the rotating screws, should be sufficient to render the polymer molten. However, the applied energy by the extrusion system should not be so great as to cause degradation of the polymer or of the other formulation components. The diameter and shape of the extruded strand is primarily governed by the diameter and geometry of the die orifice, but may also be influenced by the viscoelastic properties of the polymeric melt. Circular dies with diameters between 500 and 4000 micrometer are suitable. The extruded strands may be cut into cylindrical pellets in the hot state or after cooling to room temperature and may further be spheronized. Several technologies have been developed for the subsequent pelletization and spheronization in a continuous or semi-continuous manner and which are well-know in the art.

By means of in vitro testing it was shown that the granular pharmaceutical composition according to the present invention enhances the dispersibility of compound I in an aqueous environment due to a unique interaction between the drug particles and the at least one poloxamer. The dispersibility and dissolution of compound I from this composition was found to be independent of drug particle size. Finally, the present composition was found to enhance the oral absorption of compound I in humans with respect to a solution-based formulation concept.

In the granular pharmaceutical composition according to the present invention, particle agglomeration in aqueous media is prevented which results in enhanced bioavailability. Furthermore, the effect of food on the pharmacokinetic performance of the product is also reduced to a minimum. The preferred physical form of poloxamer 188 is fine particle material in order to enable intimate mixing. Besides poloxamer 188, other suitable poloxamers include but are not limited to poloxamer 407 and poloxamer 338. Further, other non-ionic surfactants, for instance Vitamin E TPGS (Eastman Kodak), Gelucire 44/14, Gelucire 50/13 (Gattefosse, NJ), Solutol HS15,, Lutrol F77, Cremophor RH40 (BASF, NJ), sucrose dipalmitate and sucrose distearate (Croda, NJ) can also be added.

In another embodiment, the present invention also relates to an oral dosage form comprising the granular pharmaceutical composition as described hereinbefore. The oral dosage form is preferably a tablet or capsule and may comprise additional excipients like fillers, binders, disintegrants, lubricants, anti-adherents, glidants, colorants, polymer coatings and plasticizers. The oral dosage form my further comprises an immediate release filmcoat.

Conventional tablets manufactured by common tablet compression and coating techniques require the use of several percentages of excipients in addition to the active agent(s) to optimize the physical properties of the ingredients that allow convenient manufacture of the tablet and produce a final product which is readily administered to the patient. These excipients may include fillers, binders, disintegrants, lubricants, anti-adherents, glidants, colorants, polymer coatings and plasticizers. Fillers or diluents are inert bulking agents to provide sufficient material to compress a powder into a tablet.

The membrane coating may further contain other coating excipients such as opacifiers, pigments, colorants and the like. The choice of such materials and the amounts to be utilized are considered to be within the art. In order to minimize hardening and rupture of the membrane coating, it is often desirable to utilize a plasticizer in combination with the polymeric coating material. Examples of plasticizers that can be used in accordance with the invention include: triacetin, propylene glycol, polyethylene glycol having a molecular weight of about 200 to about 1,000, dibutyl phthalate, dibutyl sebacate, triethyl citrate, vegetable and mineral oils, fatty acids, fatty acid glycerides of C₆-C₁₈ fatty acids, and the like.

The following examples illustrate the preparation of the granular pharmaceutical composition and solid dosage forms, like tablets and capsules according to the present invention. The examples and preparations hereinafter are provided to enable those skilled in the art to more clearly understand and to practice the present invention.

### Example 1

### Evaluation of dispersibility of granules containing compound I with Poloxamer 188 and PEG 8000 as binders

The granules of compound I using either poloxamer 188 or PEG 8000 as binders can be produced by hot melt extrusion. The composition of both granulation formulations is provided in Table 1. The components of these formulations can be combined using a usual powder blender. The powder blend is then hot melt extrusion processed in a commonly used twin screw extrusion system (HAAKE MiniLab) at 70°C with a screw speed of 200 RPM. The extrudate strands can then be milled using a commonly used hammermill (L1A Lab Scale FitzMill) with a 2.0 mm screen insert.

**Table 1**

| | Poloxamer Granules | PEG Granules |
|---|---|---|
| **Ingredient** | **% w/w** | **% w/w** |
| Compound I sodium salt | 40 | 40 |
| D-Mannitol pulv. | 37 | 37 |
| Poloxamer 188 | 23 | -- |
| PEG 8000 | -- | 23 |

Relative dispersibility of these granules was evaluated according to the following method:

Two grams of Compound I-poloxamer 188 and Compound I-PEG 8000 HME granules were added to separate beakers containing 250 mL of 0.1 N HCl and mixed for two minutes by magnetic stirring. After sitting for two hours without agitation, the suspensions were qualitatively assessed and photographed. The results of this analysis are shown in Fig. 2. (A) relates to Compound I-poloxamer 188 HME granules and (B) to Compound I-PEG 8000 HME granules suspended in 0.1 N HCl for two hours. Images (A) and (B) demonstrate the efficacy of poloxamer 188 with regard to maintaining a fine suspension of compound I particles in acidic media. Maintaining a finely dispersed suspension of compound I in acid is critical for improving oral absorption of compound I as it ensures that the drug is in a rapidly dissolving form when it reaches the intestinal tract. The unique interaction of compound I and poloxmer 188 in aqueous media is the underlying cause for the excellent dispersibility of these HME granules. The agglomeration that leads to the settling seen with the PEG granules is typical of formulations produced by conventional means or which to not contain at least one poloxamer.

### Example 2

### Tablet formulations of Compound I obtained by hot melt extrusion

The granulation of compound I can be achieved by a hot melt extrusion process. This is the most preferred method as it provides intimate mixing of compound I with the at least one poloxamer, preferably poloxamer 188 resulting in a more uniform and robust granular pharmaceutical composition and ultimately in the final oral dosage form. Since the hot melt extrusion process is continuous, it also provides for additional advantages in scale-up of the final oral dosage form. A typical final oral dosage form comprising a granular pharmaceutical composition in accordance with the present invention is provided in the Table 2. A corresponding manufacturing process is further schematically shown in Fig. 1.

**Table 2**

| **Ingredient** | **Amount (mg/tab)** | **%wt/wt** |
|---|---|---|
| Compound I (sodium salt) | 103.00 | 17.48 |
| D-Mannitol pulv. | 95.28 | 16.17 |
| Poloxamer 188 | 59.23 | 10.05 |
| Total Intragranular Weight | 257.51 | 43.70 |
| Mannitol (Parteck M200) | 240.34 | 40.78 |
| Croscarmellose sodium | 22.89 | 3.88 |
| Talc | 22.89 | 3.88 |
| Sodium stearyl fumarate | 22.89 | 3.88 |
| Colloidal Silicon Dioxide | 5.72 | 0.97 |
| Total Kernel Weight | 572.24 | 97.09 |
| Opadry II Brown | 17.17 | 2.91 |
| Total Tablet Weight | 589.41 | 100.00 |

The intragranular components compound I and poloxamer 188 are mixed together in a commonly used blender (bin or twin shell). The resulting powder is then fed into a commonly used extruder (American Leistritz model Micro-18 lab twin-screw extruder) using a common loss on weight feeder operated at a rate of 75 g/min while the screw rotation rate is maintained at 290 RPM. The twin screw extruder is equipped with screws of appropriate geometry for conveying and mixing the intragranular components along the barrel. The barrel consists of seven temperature controlled blocks plus the die which are maintained at the following temperatures: 35, 45, 60, 60, 60, 45, 40, 40°C. The extruded strands are transported from the die by a conveyor belt equipped with an air cooling system. The collected extrudates are then milled with a hammer mill using a 2.0 mm screen at medium speed. The granules are blended with external excipients in appropriate blender. The final blend is compressed into tablets using a tablet compression machine. The kernels can then be coated using a common film-coat in the vented coating pans.

### Example 3

### Dissolution tests of various compound I formulation concepts

Dissolution testing of the samples referenced in Fig. 3 was carried out in a SOTAX AT7 smart off-line dissolution system (SOTAX, Allschwil, Switzerland) configured with paddles (USP app. 2, rot paddle), peristaltic pump for automated sample pull and sampling station for media fill in HPLC vials. Dissolution was performed at 37°C in 900 mL 10 mM Acetate buffer pH 5.0, 10 mM Phosphate buffer pH 7.5 respectively by testing 3 or 6 units per run, applying a paddle speed of 50 RPM. Samples (1.5 mL) were pulled after 5, 10, 15, 20, 30, 45 and 60 min in a zone midway between the surface of the dissolution medium and the top of the rotating paddle but not less than 1 cm away from the vessel wall. Relevant tubings and filters were flushed with 25 mL of sample solution in closed circuit before sampling. All samples were filtered through Cannula prefilter (35 µm) or equivalent and 1 µm Glassfiber filter (e.g. Pall Acrodisc) prior to botteling for subsequent HPLC analysis.

HPLC analyses were run on a Agilent 1100 Series HPLC system or equivalent in isocratic elution mode employing UV detection at 215 nm. Pump flow rate, column temperature and injection volume were set to 1.5 mL/min, 15°C and 5 µL. Chromatographic separation was performed on a C18 reversed phase with 50 x 4.6 mm in dimension. The mobile phase consisted of a 53:47 mixture of a 20 mM Ammonium phosphate buffer pH 7.0 and acetonitrile by volume. Results were reported in % recovery referred to the specified label claim of the respective test item under investigation in consideration of the withdrawn sample volume (volume correction).

Fig. 3 shows the comparative dissolution performance of: (1) the hot-melt extruded tablets produced according to Example 1 (triangles), (2) soft gelatin capsule containing a solution of compound I (stars), and (3) a tablet containing an amorphous dispersion of compound I produced by spray drying (circles).

The dissolution results clearly demonstrate the surprisingly rapid dissolution rate of compound I from the hot-melt extruded tablets following a transition from simulated gastric fluid into pH 5.5 acetate buffer. A particularly surprising aspect of these dissolution results is that the HME tablet, which contains the drug in a substantially crystalline form, shows a similar dissolution profile to that of the soft gelatin capsule and the spray dried tablet that both contain the drug in a predominantly molecular and/or amorphous form.

Considering that compound I is a BCS 4 molecule, this result is particularly surprising because conversion of these types of drugs to an amorphous or molecular form typically results in substantial increases in dissolution rates and over the crystalline forms. Enhanced dissolution properties relate to improved pharmacokinetic performance in mammals that ultimately improves the efficacy of the molecule with respect to its therapeutic indication.

### Example 4

### Human pharmacokinetic evaluation of different compound I formulation concepts

**Table 3**

| | **Liquid filled soft capsule (reference)** | **HME formulation** |
|---|---|---|
| Cmax (ng/mL) | 35.7 (73%) | 42.7 (30%) |
| AUCO-∞ (ng*h/mL) | 40.0 (44%) | 48.1 (25%) |
| Tmax (hr) | 1.00 (0.50 - 3.00) | 1.00 (0.50 - 1.50) |
| t1/2 (hr) | 1.70 (28%) | 1.75 (29%) |

### Human plasma level Mean (CV%), except for Tmax (Median and range)

Table 3 shows that an increased bioavailability can be achieved with the granular pharmaceutical composition in accordance with the present invention and more specifically with the granular pharmaceutical composition obtained according to Example 3. This HME formulation contains compound I in undissolved, crystalline form. Surprisingly, the HME formulation shows higher AUC values with reduced variability in comparison to the liquid filled soft capsule where the compound I is already dissolved. This indicates a strong benefit due to the the intimate embedding of API in poloxamer 188 as hydrophilic polymer. The reduced variability of HME formulation leads to constant blood levels associated with reduction of potential side effects.

### Example 5

### Dog plasma level

**Table 4**

| | 100 mg compound I tablet from HME granules without Mannitol | 100 mg compound I tablet from HME granules with Mannitol |
|---|---|---|
| Cmax (ng/mL) | 658 (64) | 2760 (1180) |
| AUC (ng*h/mL) | 605 (86) | 2180 (911) |

### Dog plasma level Mean (CV)

Table 4 shows the comparison of dog plasma levels of compound I. The comparison between compound I containing tablets with and without mannitol indicates that the hydrophilic filler mannitol led to higher plasma values regarding AUC. These results indicate that in addition to poloxamer 188 also the hydrophilic filler mannitol in combination with poloxamer 188 further increases bioavailability of compound I.

### Example 6

### Dissolution testing of HME tablets produced with different particle size grades of compound I sodium salt

Dissolution testing of the samples reference in Figure 4 was carried out in a SOTAX AT7 smart off-line dissolution system (SOTAX, Allschwil, Switzerland) configured with paddles (USP app. 2, rot paddle), peristaltic pump for automated sample pull and sampling station for media fill in HPLC vials. Dissolution was performed at 37°C in 900 mL 10 mM Acetate buffer pH 5.0, 10 mM Phosphate buffer pH 7.5 respectively by testing 3 or 6 units per run, applying a paddle speed of 50 RPM. Samples (1.5 mL) were pulled after 5, 10, 15, 20, 30, 45 and 60 min. in a zone midway between the surface of the dissolution medium and the top of the rotating paddle but not less than 1 cm away from the vessel wall. Relevant tubings and filters were flushed with 25 mL of sample solution in closed circuit before sampling. All samples were filtered through Cannula prefilter (35 µm) or equivalent and 1 µm Glassfiber filter (e.g. Pall Acrodisc) prior to botteling for subsequent HPLC analysis.

HPLC analyses were run on a Agilent 1100 Series HPLC system or equivalent in isocratic elution mode employing UV detection at 215 nm. Pump flow rate, column temperature and injection volume were set to 1.5 mL/min, 15°C and 5 µL. Chromatographic separation was performed on a C18 reversed phase with 50 x 4.6 mm in dimension. The mobile phase consisted of a 53:47 mixture of a 20 mM Ammonium phosphate buffer pH7.0 and acetonitrile by volume. Results were reported in % recovery referred to the specified label claim of the respective test item under investigation in consideration of the withdrawn sample volume (volume correction).

Fig. 4 shows the results of dissolution tests comparing the release of compound I in Fig. 4(A) at pH 5.0 and Fig. 4(B) at pH 7.5 acetate buffer from tablets produced according to Example 2 containing micronized and as-is forms of compound I.

More specifically, Fig. 4 illustrates that the dissolution rate of compound I from the tablets produced according to Example 2 is independent of API particle size. The particle size distribution of compound I as obtained following the crystallization process (as-is), without further mechanical manipulation is: 1.5 µm for d(0.1), 5.0 µm for d(0.5), 34.7 µm for d(0.9). The particle size distribution of compound I as obtained by micronization of the as-is API is: 0.8 µm for d(0.1), 1.3 µm for d(0.5), 2.2 µm for d(0.9). Therefore, micronization produces a significant reduction in particle size of compound I. Conventional wisdom with regard to improving the dissolution properties of poorly water-soluble drugs states that dissolution rate increases with decreasing particle size. This is based on the Noyes-Whitney equation that demonstrates that the amount of solute mass entering the solution phase in a solvent per a given time interval is directly proportional to the surface area of the solute. By reducing particle size trough micronization the surface area of compound I is significantly increased. However, a corresponding increase in dissolution rate from the tablets produced by Example 2 is not seen when compared to as-is API.

### Example 7

### Dissolution test of compound I tablets produced with HME granules of varying particle sizes

Fig.5 shows the results of dissolution tests for compound I tablets containing hot melt extruded granules of varying size. The granules used in this study were obtained by the hot-melt extrusion and milling methods described in Example 2. The milled granules were divided by particle size by sieving. Tablets were then made from the various particle size granules. The tablets were also produced in a similar manner as Example 2.

Dissolution testing of the samples reference in Figure 5 was carried out in a SOTAX AT7 smart off-line dissolution system (SOTAX, Allschwil, Switzerland) configured with paddles (USP app. 2, rot paddle), peristaltic pump for automated sample pull and sampling station for media fill in HPLC vials. Dissolution was performed at 37°C in 900 mL 10 mM Acetate buffer pH 5.0 by testing 3 or 6 units per run, applying a paddle speed of 50 RPM. Samples (1.5 mL) were pulled after 5, 10, 15, 20, 30, 45 and 60 min. in a zone midway between the surface of the dissolution medium and the top of the rotating paddle but not less than 1 cm away from the vessel wall. Relevant tubings and filters were flushed with 25 mL of sample solution in closed circuit before sampling. All samples were filtered through Cannula prefilter (35 µm) or equivalent and 1 µm Glassfiber filter (e.g. Pall Acrodisc) prior to botteling for subsequent HPLC analysis.

HPLC analyses were run on a Agilent 1100 Series HPLC system or equivalent in isocratic elution mode employing UV detection at 215 nm. Pump flow rate, column temperature and injection volume were set to 1.5 mL/min, 15°C and 5 µL. Chromatographic separation was performed on a C18 reversed phase with 50 x 4.6 mm in dimension. The mobile phase consisted of a 53:47 mixture of a 20 mM Ammonium phosphate buffer pH7.0 and acetonitrile by volume. Results were reported in % recovery referred to the specified label claim of the respective test item under investigation in consideration of the withdrawn sample volume (volume correction).

The dissolution profiles of tablets produced with hot melt extruded granules of widely varying particle sizes are superimposable. This demonstrates that the dissolution of compound I from the tablets described herein is independent of granule size. In conventional granulation operations with poorly soluble compounds, dissolution is strongly dependant on granule particle size distribution.

The features disclosed in the foregoing description, or the following claims, expressed in their specific forms or in terms of a means for performing the disclosed function, or a method or process for attaining the disclosed result, as appropriate, may, separately, or in any combination of such features, be utilized for realizing the invention in diverse forms thereof.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

## Claims

1. A granular pharmaceutical composition comprising a compound of formula (I) 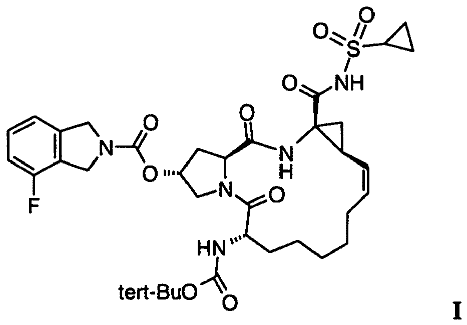 or a pharmaceutically acceptable salt thereof and at least one poloxamer, wherein the at least one poloxamer is poloxamer 188.

2. The composition according to claim 1 further comprising an intragranular filler, preferably selected from the group consisting of dicalcium phosphate, calcium sulfate, lactose, cellulose, kaolin, mannitol, sodium chloride starch and powdered sugar.

3. The composition according to claim 2 wherein the intragranular filler is mannitol, preferably in an amount of up to 80 % wt/wt, and more preferably in an amount of up to 40 % wt/wt.

4. The composition according to any one of claims 1 to 3 comprising from 20 to 50% wt/wt of the compound of formula (I) and from 20 to 40 % wt/wt of poloxamer 188.

5. The composition according to claim 4 comprising 40% wt/wt of the compound of formula (I) and 23% wt/wt of poloxamer 188.

6. The composition according to claim 5 comprising 40% wt/wt of the compound of formula (I), 23% wt/wt of poloxamer 188 and 37% wt/wt of mannitol.

7. The composition according to claim 1 being a binary composition consisting of a compound of formula (I) from 20 to 80% wt/wt and a poloxamer from 20 to 80% wt/wt, and preferably of a compound of formula (I) from 40 to 60% wt/wt and a poloxamer from 40 to 60% wt/wt.

8. The composition according to any one of claims 1 to 7 obtained by hot melt extrusion.

9. The composition according to any one of claims 1 to 8 wherein the compound of formula (I) is present in crystalline form.

10. An oral dosage form comprising the composition of any one of claims 1 to 9.

11. The oral dosage form of claim 10 further comprising at least one excipient selected from the group consisting of fillers, binders, disintegrants, lubricants, anti-adherents, glidants, colorants, polymer coatings and plasticizers.

12. The oral dosage form of claim 10 or 11 being a tablet or capsule, preferably comprising an immediate release filmcoat.

13. A composition according to any one of claims 1 to 9 for use in the treatment of HCV infections.

14. An oral dosage form according to any one of claims 10 to 12 for use in the treatment of HCV infections.

## Patentansprüche

1. Eine granuläre pharmazeutische Zusammensetzung, umfassend eine Verbindung der Formel (I) 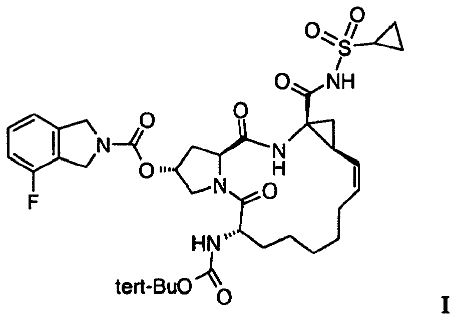 oder ein pharmazeutisch verträgliches Salz davon und mindestens ein Poloxamer, wobei das mindestens eine Poloxamer Poloxamer 188 ist.

2. Die Zusammensetzung nach Anspruch 1, weiter umfassend einen intragranulären Füllstoff, vorzugsweise ausgewählt aus der Gruppe bestehend aus Dicalciumphosphat, Calciumsulfat, Lactose, Cellulose, Kaolin, Mannitol, Natriumchloridstärke und Puderzucker.

3. Die Zusammensetzung nach Anspruch 2, wobei der intragranuläre Füllstoff Mannitol, vorzugweise in einer Menge von bis zu 80% Gew./Gew. und stärker bevorzugt in einer Menge von bis zu 40% Gew./Gew., ist.

4. Die Zusammensetzung nach einem der Ansprüche 1 bis 3, umfassend 20 bis 50% Gew./Gew. der Verbindung der Formel (I) und 20 bis 40% Gew./Gew. Poloxamer 188.

5. Die Zusammensetzung nach Anspruch 4, umfassend 40% Gew./Gew. der Verbindung der Formel (I) und 23% Gew./Gew. Poloxamer 188.

6. Die Zusammensetzung nach Anspruch 5, umfassend 40% Gew./Gew. der Verbindung der Formel (I), 23% Gew./Gew. Poloxamer 188 und 37% Gew./Gew. Mannitol.

7. Die Zusammensetzung nach Anspruch 1, bei welcher es sich um eine binäre Zusammensetzung handelt, die aus einer Verbindung der Formel (I) in 20 bis 80% Gew./Gew. und einem Poloxamer in 20 bis 80% Gew./Gew. und vorzugsweise aus einer Verbindung der Formel (I) in 40 bis 60% Gew./Gew. und einem Poloxamer in 40 bis 60% Gew./Gew. besteht.

8. Die Zusammensetzung nach einem der Ansprüche 1 bis 7, erhalten durch Schmelzextrusion.

9. Die Zusammensetzung nach einem der Ansprüche 1 bis 8, wobei die Verbindung der Formel (I) in kristalliner Form vorliegt.

10. Eine orale Dosierungsform, umfassend die Zusammensetzung nach einem der Ansprüche 1 bis 9.

11. Die orale Dosierungsform nach Anspruch 10, weiter umfassend mindestens einen Exzipienten, ausgewählt aus der Gruppe bestehend aus Füllstoffen, Bindemitteln, Sprengmitteln, Schmiermitteln, Antihaftmitteln, Gleitmitteln, Farbmitteln, Polymerüberzügen und Weichmachern.

12. Die orale Dosierungsform nach Anspruch 10 oder 11, wobei es sich um eine Tablette oder Kapsel handelt, die vorzugsweise einen Filmüberzug für die unmittelbare Freisetzung umfasst.

13. Eine Zusammensetzung nach einem der Ansprüche 1 bis 9 zur Verwendung bei der Behandlung von HCV-Infektionen.

14. Eine orale Dosierungsform nach einem der Ansprüche 10 bis 12 zur Verwendung bei der Behandlung von HCV-Infektionen.

## Revendications

1. Composition pharmaceutique granulaire comprenant un composé de formule (I) 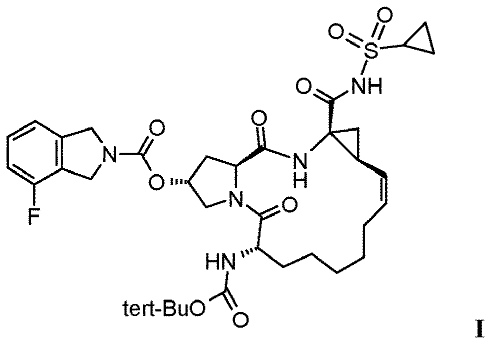 ou un sel pharmaceutiquement acceptable de celui-ci, et au moins un poloxamère, dans laquelle l'au moins un poloxamère est le poloxamère 188.

2. Composition selon la revendication 1, comprenant en outre une charge intragranulaire, de préférence choisie dans le groupe constitué par le phosphate dicalcique, le sulfate de calcium, le lactose, la cellulose, le kaolin, le mannitol, le chlorure de sodium, l'amidon, et le sucre en poudre.

3. Composition selon la revendication 2, dans laquelle la charge intragranulaire est le mannitol, de préférence en une quantité allant jusqu'à 80 % p/p, de manière plus préférée en une quantité allant jusqu'à 40 % p/p.

4. Composition selon l'une quelconque des revendications 1 à 3, comprenant de 20 à 50 % p/p du composé de formule (I) et de 20 à 40 % p/p de poloxamère 188.

5. Composition selon la revendication 4, comprenant 40 % p/p du composé de formule (I) et 23 % p/p de poloxamère 188.

6. Composition selon la revendication 5, comprenant 40 % p/p du composé de formule (I), 23 % p/p de poloxamère 188 et 37 % p/p de mannitol.

7. Composition selon la revendication 1, qui est une composition binaire constituée de 20 à 80 % p/p du composé de formule (I) et de 20 à 80 % p/p d'un poloxamère, et de préférence de 40 à 60 % p/p d'un composé de formule (I) et de 40 à 60 % p/p d'un poloxamère.

8. Composition selon l'une quelconque des revendications 1 à 7, obtenue par extrusion à l'état fondu à chaud.

9. Composition selon l'une quelconque des revendications 1 à 8, dans laquelle le composé de formule (I) est présent sous forme cristalline.

10. Forme galénique orale comprenant la composition de l'une quelconque des revendications 1 à 9.

11. Forme galénique orale selon la revendication 10, comprenant en outre au moins un excipient choisi dans le groupe constitué par les charges, les liants, les délitants, les lubrifiants, les antiadhésifs, les agents de glissements, les colorants, les enrobages polymères et les plastifiants.

12. Forme galénique orale selon la revendication 10 ou 11 qui est un comprimé ou une capsule, comprenant de préférence un enrobage pelliculaire à libération immédiate.

13. Composition selon l'une quelconque des revendications 1 à 9, pour son utilisation dans le traitement d'infection par VHC.

14. Forme galénique orale selon l'une quelconque des revendications 10 à 12, pour son utilisation dans le traitement d'infection par VHC.
